# EUROPEAN PATENT APPLICATION

(11) **EP 3 651 157 A1**
(43) Date of publication of application: **13.05.2020**
(21) Application number: 18204920.5
(22) Date of filing: 07.11.2018
(51) Int. Cl.: G16H 10/60, G16H 80/00, G16H 40/20

(54) **PROCESSING OF ANONYMISED PATIENT DATA GENERATED BY A MOBILE MEDICAL DEVICE**

(71) Applicant: Medisanté AG, 6006 Luzern (CH)
(72) Inventor: Ruch, Martin, 6006 Luzern (CH); Heiniger, Peter, 6006 Luzern (CH)
(74) Representative: SSM Sandmair

(57) **Abstract**

Disclosed is a computer-implemented method of processing medical data which is generated by a mobile patient device in an anonymised manner, sent to a cloud-based application for processing and analysing the medical data for example to observe proper functioning of the patient device. The medical data is in parallel sent to a data consumer application which personalises the medical data so as to allow patient-specific review of the information provided by the medical data by medical personnel.

## Description

### FIELD OF THE INVENTION

The present invention relates to a computer-implemented method for a computer-implemented medical method of processing medical data, corresponding executable code, a program storage medium storing such executable code and a computer for executing the executable code, as well as a medical system comprising an electronic data storage device and the aforementioned computer.

### TECHNICAL BACKGROUND

So far, patient-worn medical device for measuring the patient's condition (e.g. blood pressure) transmit the measured data via WiFi or Bluetooth connections. This, however, may be associated with a locally confined area in which the transmission can take place.

The present invention has the object of providing improved processing and management of patient data generated by a patient-worn mobile medical device.

Aspects of the present invention, examples and exemplary steps and their embodiments are disclosed in the following. Different exemplary features of the invention can be combined in accordance with the invention wherever technically expedient and feasible.

In this section, a description of the general features of the present invention is given for example by referring to possible embodiments of the invention.

In general, the invention reaches the aforementioned object by providing, in a first aspect, a computer-implemented medical method of processing medical data. The method comprises executing, on at least one processor of at least one computer (for example at least one computer being part of the navigation system), the following exemplary steps which are executed by the at least one processor.

In a (for example first) exemplary step, anonymised patient device data is transmitted from a medical device associated with a specific patient to a server (e.g. a gateway server which functions as a gateway to the below-described cloud-based data transfer application), wherein the medical device comprises a memory and a mobile wireless data transfer module and a SIM card storing an unique identifier for the SIM card (i.e. a unique identifier which identifies the SIM card). The medical device is a mobile device which is for example attached to the patient during the patient's daily activities and which is powered by a battery installed in the medical device. Alternatively or additionally, the unique identifier identifies the medical device. The mobile wireless data transfer module is for example a GSM (Global System for Mobile communications) module usable for mobile telecommunication. The anonymised patient device data is transmitted from the medical device to the server via the mobile wireless data transfer module. The anonymised patient device data describes patient state information and device information, the device information including the unique identifier. The patient state information is for example stored in the memory of the medical device. In examples, the patient state information includes information about a medical state, for example a pathologic state, and/or at least one physiological value of the patient. The physiological value has for example been measured on the patient by the medical device and describes for example at least one of the following:
- blood pressure information by defining for example the systolic and diastolic blood pressure values together with the heartbeat of the patient;
- blood glucose level information by defining the blood glucose value of the patient;
- the patient's body weight by defining the body weight value of the patient.
The patient device data being anonymised means that it cannot be used to identify, without further knowledge, the identity of a patient from whom the patient device data has been generated, for example measured. In examples, the device information is information about a value of an operating parameter of the medical device which describes for example at least one of the following:
- a signal strength which the medical device receives from the radio infrastructure (i.e. mobile telecommunication network);
- voltage level of the battery installed in the medical device (i.e. total or remaining battery lifetime).
In one example, the device information comprises a token for authenticating the anonymised patient device data. For example, the token is valid for only a predetermined number of transmissions or certain timeframe, for example one transmission or one month, of the anonymised patient device data from the patient device to the server. This means that the token in this example is renewed after each transmission or expiry of a certain time interval, thereby increasing transmission security. In one example, the data transfer application sends, after receiving the patient device data from the medical device, a command to the medical device for deletion of the patient state information from the memory of the medical device. The patient state information is consequently deleted from the memory of the medical device which serves improved management of the memory installed in the medical device.

In a (for example second) exemplary step, the anonymised patient device data is input, by the server, to a cloud-based data transfer application comprising an input adapter, a data queue and an output adapter. The data transfer application being cloud-based means that it is configured to be executed (for example, is executed) in the cloud, i.e. on at least one cloud server.

In a (for example third) exemplary step, the anonymised patient device data is analysed by the input adapter whether it has a data format processable by the data transfer application. If the result of the analysis is that the anonymised patient device data does not have a format processable by the data transfer application, the anonymised patient device data is re-formatted by the input adapter into a format processable by the data transfer application.
In a (for example fourth) exemplary step which takes place after the analysis by the input adapter, the anonymised patient device data is re-formatted by the input adapter if the result of the analysis is that the anonymised patient device data does not have a format processable by the data transfer application, and then transmitted from the data transfer application to the device management application. The device management application then determines, by accessing a mobile wireless data transfer server managing the transmission of the anonymised patient device data from the medical device, whether the anonymised patient device data was transmitted to the server via the mobile wireless data transfer module by comparing the unique identifier included in the device information to a history of mobile wireless data transfer connections managed by the mobile wireless data transfer server. This constitutes a data security and/or integrity check to ensure that the patient device data was transmitted via the correct route and not for example maliciously sent from an undesired source. If the result of the comparison is that anonymised patient device data was not transmitted to the server via the mobile wireless data transfer module, the device managing application issues a corresponding notification. This constitutes a security warning in case the integrity check raises for example a suspicion of hacking of the transmission route. If the result of the comparison is that anonymised patient device data was transmitted to the server via the mobile wireless data transfer module (i.e. if there is no suspicion of hacking on the basis of the result of the comparison), the anonymised patient device data is stored in the data queue.

In a (for example fifth) exemplary step, the anonymised patient device data is analysed by the output adapter whether it has a data format processable by the data consumer application. If the result of the analysis is that the anonymised patient device data does not have a format processable by the data consumer application, the anonymised patient device data is re-formatted by the output adapter into a format processable by the data consumer application.

In a (for example sixth) exemplary step which is executed after the analysis by the output adapter and, if the result of the analysis is that the anonymised patient device data does not have a format processable by the data consumer application, the anonymised patient device data is re-formatted by the output adapter. The anonymised patient device data is then transmitted from the data transfer application to the data consumer application by either sending a request, from a data consumer application to the data transfer application, for transmission of the anonymised patient device data stored in the cloud-based data transfer application, or pushing, by the data transfer application and based on routing information stored in the device management application, the anonymised patient data to the data consumer application. The patient device data is then for example personalised (i.e. assigned to the patient so that the patient's identity is, visibly to a user such as medical personnel, associated with the patient device data) for example by the data consumer application. In one example, this is done by comparing the unique identifier of the SIM card and/or the medical device to unique identifiers stored in a database and/or look-up table in (for example unique) association with patient identities. The patient device data is then assigned to the patient identity which is associated with the unique identifier included in the device information.

In an example of the method according to the first aspect, the device managing application analyses the device information and sends a notification or updated configuration data to at least one of the medical device or the data consumer application in dependence on the result of analysing the device information.

In an additional or alternative example of the method according to the first aspect, the medical device sends routine check messages over the data transfer application to the device managing application at predetermined time intervals. This constitutes a pseudo-ping for checking whether the medical device can still be reached. Such a check is for example executed by the device management application, and if it results in that the medical cannot be reached, a corresponding message can be output to the medical device (e.g. for alerting the patient) and/or the data consumer application (e.g. for alerting medical personnel).

In a second aspect, the invention is directed to executable code, for example a program, which is executable by a computer and which, when running on a computer or when loaded onto a computer, causes the computer to perform the method steps of the above-described method according to the first aspect. The invention may alternatively or additionally relate to a (physical, for example electrical, for example technically generated) signal wave, for example a digital signal wave, such as an electromagnetic carrier wave carrying information which represents the program, for example the aforementioned program, which for example comprises code means which are adapted to perform any or all of the steps of the method according to the first aspect. A computer program stored on a disc is a data file, and when the file is read out and transmitted it becomes a data stream for example in the form of a (physical, for example electrical, for example technically generated) signal. The signal can be implemented as the signal wave, for example as the electromagnetic carrier wave which is described herein. For example, the signal, for example the signal wave is constituted to be transmitted via a computer network, for example LAN, WLAN, WAN, mobile network, for example the internet. For example, the signal, for example the signal wave, is constituted to be transmitted by optic or acoustic data transmission. The invention according to the second aspect therefore may alternatively or additionally relate to a data stream representative of the aforementioned executable code.

In a third aspect, the invention is directed to a for example non-transitory computer-readable program storage medium on which the executable code according to the second aspect is stored.

In a fourth aspect, the invention is directed to a set of computers, for example at least or exactly one computer (for example, a computer) or a plurality of computers, each computer comprised in the set of computers comprising at least one processor (for example, a processor) and at least one memory (for example, a memory), wherein the executable code according to the second aspect is running on the processor or is loaded into the memory, or wherein the at least one computer comprises the computer-readable program storage medium according to the third aspect.

In a fifth aspect, the invention is directed to a system for processing medical data, comprising:
a) the set of computers according to the fourth aspect;
b) the medical device;
c) the server;
d) at least one electronic data storage device storing the data transfer application and the device managing application; and
e) the data consumer application,
wherein the set of computers is operably coupled to the at least one electronic data storage device for controlling the processing of the data transfer application and the device managing application

In a sixth aspect, the invention is directed to a for example non-transitory computer-readable program storage medium storing a program for causing the computer according to the fourth aspect to execute the data processing steps of the method according to the first aspect.

In a seventh aspect, the invention is directed to use of the system according to the fifth aspect for processing medical data, wherein the use comprises execution of the steps of the method according to the first aspect for managing the anonymised patient device data.

For example, the invention does not involve or in particular comprise or encompass an invasive step which would represent a substantial physical interference with the body requiring professional medical expertise to be carried out and entailing a substantial health risk even when carried out with the required professional care and expertise.

For example, the invention does not comprise a step of invasively measuring a physiological parameter on the patient. More particularly, the invention does not involve or in particular comprise or encompass any surgical or therapeutic activity. The invention is instead directed as applicable to digital data processing. For this reason alone, no surgical or therapeutic activity and in particular no surgical or therapeutic step is necessitated or implied by carrying out the invention.

### DEFINITIONS

In this section, definitions for specific terminology used in this disclosure are offered which also form part of the present disclosure.

The method in accordance with the invention is for example a computer implemented method. For example, all the steps or merely some of the steps (i.e. less than the total number of steps) of the method in accordance with the invention can be executed by a computer (for example, at least one computer). An embodiment of the computer implemented method is a use of the computer for performing a data processing method. An embodiment of the computer implemented method is a method concerning the operation of the computer such that the computer is operated to perform one, more or all steps of the method.

The computer for example comprises at least one processor and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating or determining steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term computer includes a server resource. The term "cloud computer" includes a cloud computer system which for example comprises a system of at least one cloud computer and for example a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (laaS). The cloud computer can function as a virtual host for an operating system and/or data processing application which is used to execute the method of the invention. The cloud computer is for example an elastic compute cloud (EC2) as provided by Amazon Web Services™. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing (medical) imaging methods), wherein the technical signals are for example electrical or optical signals. The technical signals for example represent the data received or outputted by the computer. The computer is preferably operatively coupled to a display device which allows information outputted by the computer to be displayed, for example to a user. One example of a display device is a virtual reality device or an augmented reality device (also referred to as virtual reality glasses or augmented reality glasses) which can be used as "goggles" for navigating. A specific example of such augmented reality glasses is Google Glass (a trademark of Google, Inc.). An augmented reality device or a virtual reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. The monitor may also be the monitor of a portable, for example handheld, device such as a smart phone or personal digital assistant or digital media player.

The invention also relates to a program which, when running on a computer, causes the computer to perform one or more or all of the method steps described herein and/or to a program storage medium on which the program is stored (in particular in a non-transitory form) and/or to a computer comprising said program storage medium and/or to a (physical, for example electrical, for example technically generated) signal wave, for example a digital signal wave, such as an electromagnetic carrier wave carrying information which represents the program, for example the aforementioned program, which for example comprises code means which are adapted to perform any or all of the method steps described herein.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer; a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is preferably a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

The expression "acquiring data" for example encompasses (within the framework of a computer implemented method) the scenario in which the data are determined by the computer implemented method or program. Determining data for example encompasses measuring physical quantities and transforming the measured values into data, for example digital data, and/or computing (and e.g. outputting) the data by means of a computer and for example within the framework of the method in accordance with the invention. A step of "determining" as described herein for example comprises or consists of issuing a command to perform the determination described herein. For example, the step comprises or consists of issuing a command to cause a computer, for example a remote computer, for example a remote server, for example in the cloud, to perform the determination. Alternatively or additionally, a step of "determination" as described herein for example comprises or consists of receiving the data resulting from the determination described herein, for example receiving the resulting data from the remote computer, for example from that remote computer which has been caused to perform the determination. The meaning of "acquiring data" also for example encompasses the scenario in which the data are received or retrieved by (e.g. input to) the computer implemented method or program, for example from another program, a previous method step or a data storage medium, for example for further processing by the computer implemented method or program. Generation of the data to be acquired may but need not be part of the method in accordance with the invention. The expression "acquiring data" can therefore also for example mean waiting to receive data and/or receiving the data. The received data can for example be inputted via an interface. The expression "acquiring data" can also mean that the computer implemented method or program performs steps in order to (actively) receive or retrieve the data from a data source, for instance a data storage medium (such as for example a ROM, RAM, database, hard drive, etc.), or via the interface (for instance, from another computer or a network). The data acquired by the disclosed method or device, respectively, may be acquired from a database located in a data storage device which is operably to a computer for data transfer between the database and the computer, for example from the database to the computer. The computer acquires the data for use as an input for steps of determining data. The determined data can be output again to the same or another database to be stored for later use. The database or database used for implementing the disclosed method can be located on network data storage device or a network server (for example, a cloud data storage device or a cloud server) or a local data storage device (such as a mass storage device operably connected to at least one computer executing the disclosed method). The data can be made "ready for use" by performing an additional step before the acquiring step. In accordance with this additional step, the data are generated in order to be acquired. The data are for example detected or captured (for example by an analytical device). Alternatively or additionally, the data are inputted in accordance with the additional step, for instance via interfaces. The data generated can for example be inputted (for instance into the computer). In accordance with the additional step (which precedes the acquiring step), the data can also be provided by performing the additional step of storing the data in a data storage medium (such as for example a ROM, RAM, CD and/or hard drive), such that they are ready for use within the framework of the method or program in accordance with the invention. The step of "acquiring data" can therefore also involve commanding a device to obtain and/or provide the data to be acquired. In particular, the acquiring step does not involve an invasive step which would represent a substantial physical interference with the body, requiring professional medical expertise to be carried out and entailing a substantial health risk even when carried out with the required professional care and expertise. In particular, the step of acquiring data, for example determining data, does not involve a surgical step and in particular does not involve a step of treating a human or animal body using surgery or therapy. In order to distinguish the different data used by the present method, the data are denoted (i.e. referred to) as "XY data" and the like and are defined in terms of the information which they describe, which is then preferably referred to as "XY information" and the like.

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Fig. 1: illustrates a basic flow of the method according to the first aspect;
- Fig. 2: shows a data processing architecture of an embodiment for executing the method according to the first aspect;
- Fig. 3: illustrates personalisation of the anonymised patient device data; and
- Fig. 4: is a schematic illustration of the system according to the fifth aspect.

### DESCRIPTION OF EMBODIMENTS

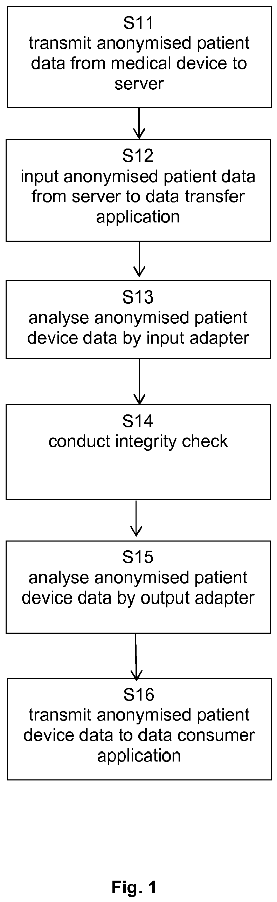

Figure 1 illustrates the basic steps of the method according to the first aspect, in which step S11 encompasses transmission of the anonymised patient data from the medical device to the server, step S12 encompasses the input of the anonymised patient data from the server to the data transfer application and subsequent step S13 encompasses analysis of the anonymised patient data by the input adapter and any necessary re-formatting. This is followed by step S14 of conducting the integrity check to become aware of malicious data transmission. Then, the anonymised patient data is analysed by the output adapter and, if necessary, re-formatted in step S15. In subsequent step S16, the anonymised patient data is transmitted from the data transfer application to the data consumer application.

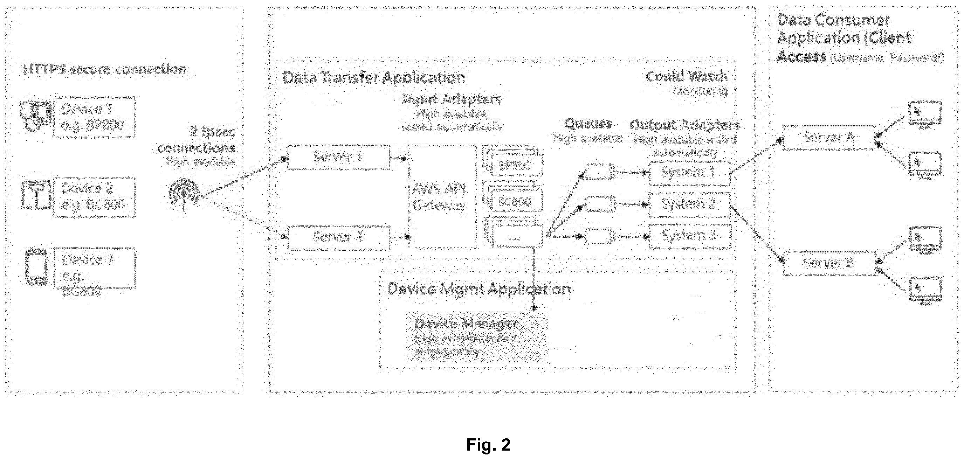

Figure 2 describes the architecture of a specific implementation of the system according to the fifth aspect for executing the method according to the first aspect. At least one devices (device 1, device 2, or device 3) sends via a HTTP (hypertext transfer protocol) secure connection the anonymised patient device data for example via 2 Ipsec connections to at least one of the gateway server (server 1 and server 2) to the input adapter included for example in an Amazon Web Service™ (AWS) application programming interface (API) gateway and is from there distributed to the device management application (designated "Device Mgmt Application" in Fig. 2). Additionally, the anonymised patient data is fed into the queues and from thereon to the output adapter which conducts any potentially necessary re-formatting of the anonymised patient device data to the requirements of data consumer application running on at least one terminal to which the anonymised patient device data is transmitted from the data transfer application via at least one server (server A, server B) and by which it is then personalised.

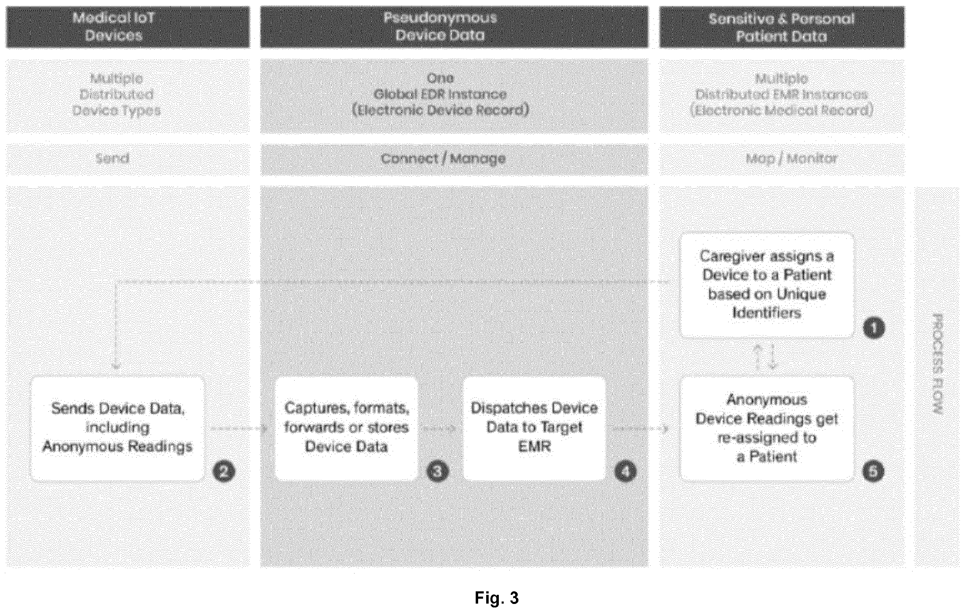

Figure 3 shows the systematic division of the architecture of Fig. 2 into a sphere in which the patient device data is anonymised and a sphere in which the patient device data is personalised. Multiple different types of medical loT (Internet of Things) devices send the patient device data including the anonymised patient state information to the data transfer application which processes the patient device data to conform to the technical requirements of the subsequent data processing and connects the medical loT devices with the electronic medical record stored at the data consumer application end. The data consumer application assigns the patient device data to the identity of a patient and thereby becomes personalised. The unique identifier used to do so has beforehand been assigned to the patient by medical personnel ("caregiver") and stored in a look-up table to allow personalising the patient device data.

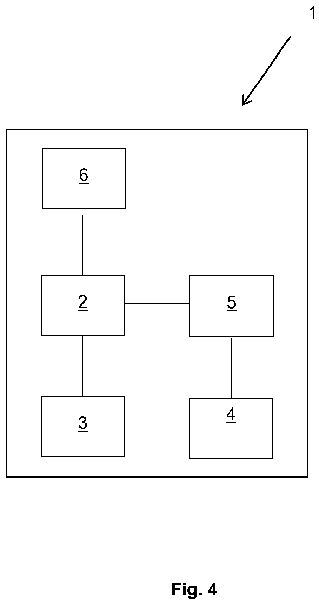

Figure 4 is a schematic illustration of the medical system 1 according to the fifth aspect. The system is in its entirety identified by reference sign 1 and comprises a set of computers 2, an electronic data storage device (such as a hard disc) 3 for storing at least the data transfer application and the device managing application and a medical device 4 (such as a glucometer or blood pressure and/heart beat measuring device). Further components of the system 1 are the server 5 and the data consumer application 6. The components of the medical system 1 have the functionalities and properties explained above with regard to the fifth aspect of the invention.

The present inventions also has the following features:

### A hub-and-spoke architecture

The present invention allows delivering a connected care service through the combination of
- cellular medical devices covering multiple conditions;
- a global connected care platform implemented as the data transfer application; and
- a SaaS-based (Software-as-a-Service-based) EMR (electronic medical record) natively designed for cellular loT.
A hub and spoke architecture around the data transfer application allows seamlessly connecting multiple cellular device types with multiple Electronic Medical Record (EMR) instances.

### Delivering both RPM and RDM

Telemonitoring requires both RPM (Remote Patient Monitoring) and RDM (Remote Device Management) in order to scale. The present invention covers it both while any other solution based on bluetooth or Wi-Fi devices will leave the biomedical engineer blind in the last meter from the modem to the patient.

### Openness and simplicity

Openness and simplicity are at the core of the cellular approach to loT that the invention heralds:
- The direct inclusion of a SIM-card in each medical device removes the need for any clunky bluetooth or Wi-Fi connection on the patient-side to transmit data.
- Connecting bio-medical teams with technical device attributes and allowing them to centrally manage the device inventory radically simplifies the deployment of large scale medical loT projects.
- Caregivers are the ones who assign home-care devices to individual patients within an EMR subject to strict privacy and healthcare regulations like they are used to do with hospital equipment.
- The sensor-readings sent over the M2M cellular network are encrypted pseudonymous device data (within the framework of this disclosure, called "anonymised") until they get mapped back to the patient in an EMR.

In summary, the present invention is directed to a computer-implemented method of processing medical data which is generated by a mobile patient device in an anonymised manner, sent to a cloud-based application for processing and analysing the medical data for example to observe proper functioning of the patient device. The medical data is in parallel sent to a data consumer application which personalises the medical data so as to allow patient-specific review of the information provided by the medical data by medical personnel.

## Claims

1. A computer-implemented method of processing medical data, the method comprising the following steps:
a) anonymised patient device data is transmitted (S11) from a medical device associated with a specific patient to a server, wherein the medical device comprises a memory and a mobile wireless data transfer module and a SIM card storing an unique identifier for the SIM card and/or the medical device, wherein the anonymised patient device data is transmitted from the medical device to the server via the mobile wireless data transfer module, and wherein the anonymised patient device data describes patient state information and device information, the device information including the unique identifier, the patient state information being stored in the memory of the medical device;
b) the anonymised patient device data is input (S12), by the server, to a cloud-based data transfer application comprising an input adapter, a data queue and an output adapter;
c) the anonymised patient device data is analysed (S13) by the input adapter whether it has a data format processable by the data transfer application. If the result of the analysis is that the anonymised patient device data does not have a format processable by the data transfer application, the anonymised patient device data is re-formatted by the input adapter into a format processable by the data transfer application;
d) after the analysis by the input adapter and, if the result of the analysis is that the anonymised patient device data does not have a format processable by the data transfer application, re-formatting the anonymised patient device data by the input adapter, the anonymised patient device data is transmitted from the data transfer application to the device management application, and the device management application then determines (S14), by accessing a mobile wireless data transfer server managing the transmission of the anonymised patient device data from the medical device, whether the anonymised patient device data was transmitted to the server via the mobile wireless data transfer module by comparing the unique identifier included in the device information to a history of mobile wireless data transfer connections managed by the mobile wireless data transfer server, and
α) if the result of the comparison is that anonymised patient device data was not transmitted to the server via the mobile wireless data transfer module, the device managing application issues a corresponding notification; and
β) if the result of the comparison is that anonymised patient device data was transmitted to the server via the mobile wireless data transfer module, the anonymised patient device data is stored in the data queue;
e) the anonymised patient device data is analysed (S15) by the output adapter whether it has a data format processable by the data consumer application, and if the result of the analysis is that the anonymised patient device data does not have a format processable by the data consumer application, the anonymised patient device data is re-formatted by the output adapter into a format processable by the data consumer application;
f) after the analysis by the output adapter and, if the result of the analysis is that the anonymised patient device data does not have a format processable by the data consumer application, re-formatting the anonymised patient device data by the output adapter, the anonymised patient device data is transmitted (S16) from the data transfer application to the data consumer application by
α) either sending a request, from a data consumer application to the data transfer application, for transmission of the anonymised patient device data stored in the cloud-based data transfer application;
β) or pushing, by the data transfer application and based on routing information stored in the device management application, the anonymised patient data to the data consumer application.

2. The method according to the preceding claim, wherein the patient device data is personalised by the data consumer application by comparing the unique identifier of the SIM card and/or the medical device to unique identifiers stored in a database and/or look-up table in association with patient identities, and assigning the patient device data to the patient identity which is associated with the unique identifier included in the device information.

3. The method according to any one of the preceding claims, wherein the device information comprises a token for authenticating the anonymised patient device data.

4. The method according to the preceding claim, wherein the token is valid for only a predetermined number of transmissions or certain timeframe, for example one transmission or one month, of the anonymised patient device data from the patient device to the server.

5. The method according to any one of the preceding claims, wherein the patient state information includes information about a medical state, for example a pathologic state, and/or at least one physiological value of the patient.

6. The method according to any one of the preceding claims, wherein the data transfer application sends a command to the medical device for deletion of the patient state information from the memory of the medical device.

7. The method according to any one of the preceding claims, wherein the device information is information about a value of an operating parameter of the medical device.

8. The method according to the preceding claim, wherein the device managing application analyses the device information and sends a notification or updated configuration data to at least one of the medical device or the data consumer application in dependence on the result of analysing the device information.

9. The method according to any one of the preceding claims, wherein the medical device sends routine check messages over the data transfer application to the device managing application at predetermined time intervals.

10. Executable code, for example a program, which is executable by a computer and which, when running on a computer or when loaded onto a computer, causes the computer to perform the method steps of the method according to any one of the preceding claims.

11. A computer-readable program storage medium on which the executable code according to the preceding claim is stored.

12. A set of computers, each computer of the set of computers comprising at least one processor and a memory and/or the program storage medium according to the preceding claim, wherein the executable code according to claim 9 is running on the set of computers or loaded into the memory of at least one computer of the set of computers.

13. An electromagnetic carrier wave carrying information which represents the executable code according to claim 9.

14. A data stream which is representative of the executable code according to claim 9.

15. A system (1) for processing medical data, comprising:
a) the set of computers (2) according to the preceding claim;
b) the medical device (4); and
c) the server (5);
d) at least one electronic data storage device (3) storing the data transfer application and the device managing application; and
e) the data consumer application (6),
wherein the set of computers (2) is operably coupled to the at least one electronic data storage device (3) for controlling the processing of the data transfer application and the device managing application.
